# EUROPÄISCHE PATENTSCHRIFT

(11) **EP 3 883 637 B1**
(45) Veröffentlichungstag und Bekanntmachung des Hinweises auf die Patenterteilung: **24.07.2024**
(21) Anmeldenummer: 19808726.4
(22) Anmeldetag: 19.11.2019
(51) Int. Cl.: A61M 37/00, A61N 5/10, A61M 5/32, A61M 25/06

(54) **VORRICHTUNG ZUM ABLEGEN EINES ELEMENTES MIT EINER KANÜLE**
DEVICE FOR DEPOSITING AN ELEMENT BY MEANS OF A CANNULA
DISPOSITIF SERVANT À DÉPOSER UN ÉLÉMENT POURVU D'UNE CANULE

(30) Priorität: 23.11.2018 DE 102018129618
(43) Veröffentlichungstag der Anmeldung: 29.09.2021
(73) Patentinhaber: sfm medical devices GmbH, 63607 Wächtersbach (DE)
(72) Erfinder: RICHTER, Timo, 63589 Linsengericht (DE); BRÖMSEN, Olaf, 64546 Mörfelden (DE)
(74) Vertreter: Stoffregen, Hans-Herbert
(86) Internationale Anmeldenummer: PCT/EP2019/081754
(87) Internationale Veröffentlichungsnummer: WO 2020/104430

(56) Entgegenhaltungen:
- WO-A1-2013/167431
- DE-A1- 102007 021 243
- US-A- 5 312 345
- US-A- 5 810 769
- US-A1- 2009 131 908
- US-A1- 2015 297 213

## Beschreibung

Die Erfindung bezieht sich auf eine Vorrichtung, insbesondere zum Ablegen eines Elementes, wie Feststoffmedikament oder Implantat, umfassend eine Kanüle mit am distalen Ende vorhandener Spitze und einem den proximalen Bereich der Kanüle aufnehmenden Ansatz, der vorzugsweise zumindest eine quer, insbesondere senkrecht, zur Längsachse der Kanüle verlaufende Rippe aufweist, sowie einen zumindest abschnittsweise innerhalb der Kanüle verschiebbaren Kolben mit einer Handhabe.

In der Human- und Veterinärmedizin werden mittels Injektionsvorrichtungen Feststoffmedikamente oder Implantate abgelegt, bei denen es sich um Markierungen handeln kann, um z. B. Tiere zu identifizieren bzw. überwachen zu können. Entsprechende Vorrichtungen sind z. B. aus der WO 2013/167431 A1 oder der EP 2 314 342 A1 bekannt. Dabei besteht zum Schutz der die Elemente applizierenden Personen die Möglichkeit, den auch als Mandrin zu bezeichnenden Kolben nach Ablegen des Elementes zu fixieren, d. h., dass die Kanülenspitze durch den Kolben abgedeckt ist, so dass eine Verletzungsgefahr ausgeschlossen ist.

Der DE 10 2012 104 058 A1 ist eine Kanüle mit verschiebbar angeordnetem Mandrin zu entnehmen, von dem ein Rasthaken ausgeht, der in einer Aussparung des Kanülenhalters eingreift, wenn das distale Ende des Mandrins die Spitze der Kanüle abdeckt.

Gegenstand der DE 10 2013 112 324 A1 ist eine Kanüle mit einem Mandrin, der durch Wechselwirken mit dem Schliff der Kanüle lagefixiert wird.

Ein Punktionsinstrument ist aus der US 2017/0065805 A1 bekannt, bei der ein Mandrin die Spitze eines Tubus überragt.

Der US 2009/0131908 A1 ist eine Vorrichtung zum Ablegen von Elementen zu entnehmen, die eine Kanüle und einen in dieser verschiebbaren Kolben zum Ausstoßen der Elemente aufweist. Der Kolben weist eine Handhabe mit Außengewinde auf, das in ein Innengewinde eines Ansatzes einschraubbar ist, von dem die Kanüle ausgeht.

Der vorliegenden Erfindung liegt u.a. die Aufgabe zu Grunde, eine Vorrichtung der eingangs genannten Art so weiterzubilden, dass dann, wenn eine Nutzung nicht mehr erfolgen soll, ein Schutz gegen Verletzungen durch die Kanülenspitze gegeben ist. Nach einem weiteren Aspekt soll die Möglichkeit bestehen, die Vorrichtung mehrfach zum Ablegen von Elementen zu nutzen.

Auch soll nach einem weiteren Aspekt der Erfindung für die Nutzer problemlos erfassbar sein, ob der Kolben in eine Position bewegt wird oder bewegt worden ist, in der die Spitze abgedeckt werden soll bzw. abgedeckt ist.

Zur Lösung zumindest einer der Aspekte sieht die Erfindung im Wesentlichen vor, dass die Handhabe des Kolbens einen Basisabschnitt mit von diesem ausgehenden sich in Längsrichtung des Kolbens und beabstandet zu diesem erstreckenden stegartigen Halteelementen aufweist, dass jedes Halteelement mit dem Kolben über ein Verbindungselement verbunden ist, das zumindest in einem seiner Endbereiche durch Wechselwirken mit dem Ansatz durchtrennbar ist. Insbesondere ist vorgesehen, dass das Verbindungselement durchtrennbar mit dem Halteelement und gelenkig mit dem Kolben verbunden ist oder umgekehrt.

Durch eine diesbezügliche Konstruktion der zum Verschieben des Kolbens benutzten Handhabe besteht zum einen die Möglichkeit, dass die auch als Zungen zu bezeichnenden Halteelemente mit den mit dem Kolben verbindenden Verbindungselementen einerseits als Anschlag wirken, so dass eine mehrfache Nutzung erfolgen kann, andererseits jedoch bei Krafteinwirkung auf die Handhabe in Richtung des distalen Bereichs der Kanüle von dem Halteelement getrennt werden, wobei bei weiterem Verschieben des Kolbens die Halteelemente aufgrund der weiterhin bestehenden Verbindung, insbesondere gelenkigen Verbindung mit dem Kolben durch Wechselwirken mit dem Ansatz entlang des Kolbens ausgerichtet werden, so dass diese im Bereich der Applikation nicht zur Gefährdung führen können. Gleichzeitig erfolgt ein Verrasten dadurch, dass die quasi hakenförmig ausgebildeten Halteelemente mit einer Rastaufnahme, insbesondere der ersten Rippe, des Ansatzes wechselwirken und diese hintergreifen, so dass ein unkontrolliertes Herausziehen des Kolbens aus der Kanüle ausgeschlossen ist. Folglich ist deren Spitze bleibend durch den Kolben abgedeckt.

Dabei ist insbesondere vorgesehen, dass das Halteelement distal einen in Richtung der Kolbenstange sich erstreckenden Rastabschnitt aufweist, so dass sich eine hakenförmige Geometrie ergibt.

Insbesondere ist vorgesehen, dass das Verbindungselement mit dem Rastabschnitt verbunden ist.

Ist bevorzugterweise vorgesehen, dass die Verbindungselemente gelenkig mit dem Kolben verbunden sind, so besteht selbstverständlich auch die Möglichkeit, dass eine gelenkige Verbindung mit den Halteelementen gegeben ist, wobei die Verbindungselemente von dem Kolben trennbar sind.

Die Erfindung sieht auch vor, dass die Verbindungselemente an beiden Enden, das heißt einerseits im Verbindungsbereich zu den Halteelementen und andererseits zu dem Kolben durchtrennbar sind.

Um ein sicheres und problemloses Trennen des Verbindungselements von dem Rastelement zu ermöglichen, sieht die Erfindung des Weiteren vor, dass der Ansatz proximal einen Hohlzylinderabschnitt mit einem Außendurchmesser D aufweist und dass lichter Abstand von zwei in Bezug auf die Kolbenstangenlängsachse diametral gegenüberliegenden Rastabschnitten gleich oder in etwa gleich D ist. Somit wirkt beim Verschieben des Kolbens in Richtung des Ansatzes der Hohlzylinderabschnitt unmittelbar auf den jeweiligen Verbindungsbereich zwischen dem Verbindungselement und dem Halteelement ein, so dass ein problemloses Trennen möglich ist. Die gegenüberliegende Seite ist über ein Filmscharnier mit dem Kolben selbst verbunden, so dass bei weiterer Bewegung des Kolbens in die Kanüle hinein die Verbindungselemente sich an den Kolben anlegen und in den Zwischenraum zwischen dem Hohlzylinderabschnitt und dem Kolben gelangen.

Ein selbstständiger Lösungsvorschlag sieht vor, dass die Handhabe mit dem Ansatz über eine Schraubverbindung verbindbar ist und Länge des Kolbens auf Länge der Kanüle derart abgestimmt ist, dass in einer ersten Position, in der bei fehlender Verschraubung, jedoch Kontaktierung des Ansatzes und der Handhabe die Spitze von dem Kolben unbedeckt ist, und dass in einer zweiten Position der Ansatz durch Hineinschrauben der Handhabe in den Ansatz und Überwinden eines Arretierelements verrastet sind, wobei der distale Endbereich des Kolbens die Spitze zur Unterbindung einer Verletzungsgefahr durch die Spitze abdeckt und eine entgegengesetzte Schraubbewegung ausgeschlossen ist.

Dabei zeichnet sich die Erfindung insbesondere dadurch aus, dass in der zweiten Position der Ansatz und die Handhabe verrastet sind. Das Verrasten kann dabei derart gestaltet sein, dass dieses haptisch und/oder akustisch wahrnehmbar ist.

Um Handhabe und Ansatz zu verschrauben, besteht die Möglichkeit, dass die Handhabe ein Außengewinde aufweist, das mit einem im Ansatz vorhandenen Innengewinde wechselwirkt.

Alternativ sieht die Erfindung vor, dass die Handhabe ein Innengewinde aufweist, das entweder mit von dem Ansatz, insbesondere von dessen proximalem Randbereich abragenden Stegen, wie Luer-Lock-Stege, oder mit einem Außengewinde des Ansatzes wechselwirkt.

Der Basisabschnitt der Handhabe des Kolbens weist vorzugsweise umfangsseitig eine zylinderförmige Geometrie auf. Dabei ist insbesondere vorgesehen, dass die Umfangswandung des Basisabschnitts im Mittenbereich einen geringeren Durchmesser als in seinen Endbereichen aufweist, so dass ein problemloses Erfassen möglich ist.

Die Halteelemente können außenseitig fluchtend in die Umfangsfläche des Basisabschnitts übergehen.

Die Handhabe selbst kann ein Kunststoffspritzgussteil sein.

Durch die erfindungsgemäßen Lehren soll abhängig davon, ob mit der Kanüle ein Feststoffmedikament oder Implantat oder nacheinander mehrere entsprechende Elemente abgelegt werden sollen, sichergestellt werden, dass nach Nutzung der Kanüle, also nach dem Entfernen aus einem Lebewesen, die Kanülenspitze derart abgedeckt ist, dass eine Verletzungsgefahr unterbunden ist.

Dabei können Ansatz der Kanüle und Handhabe des Kolbens, der auch als Mandrin zu bezeichnen ist, konstruktiv derart ausgebildet sein, dass zwei definierte Stellungen von Anschlag und Ansatz haptisch und/oder akustisch wahrnehmbar sind.

Bei die Kanülenspitze nicht abdeckender Position des Kolbens wirkt ein Anschlag gegen ein weiteres Verschieben des Kolbens in Richtung der Kanülenspitze. Dieser Anschlag kann durch die Verbindungselemente zwischen den Halteelementen und dem Kolben gebildet sein.

Alternativ ist der Anschlag durch Zusammenwirken von Handhabe und Anschlag vor deren Verschraubung miteinander gegeben. Soll die Kanülenspitze abgedeckt werden, wird der Anschlag überwunden und insbesondere ein Verrasten von Ansatz und Handhabung in einer zweiten vorgegebenen Position erfolgt, sei es durch Wechselwirken der Halteelemente mit Rastelementen wie eine Rippe des Ansatzes, sei es durch Überwinden eines Vorsprungs wie Absatz beim Verschrauben.

Weitere Einzelheiten, Vorteile und Merkmale der Erfindung ergeben sich nicht nur aus dem Ansprüchen, den diesen zu entnehmenden Merkmalen - für sich und/oder in Kombination-, sondern auch aus der nachfolgenden Beschreibung eines der Zeichnung zu entnehmenden bevorzugten Ausführungsbeispiels.

Es zeigen:
- Fig. 1: eine Kanüle mit Schutzhülle und Kolben im Anlieferungszustand,
- Fig. 2: die Kanüle mit Kolben gemäß Fig. 1, jedoch mit entfernter Schutzhülle,
- Fig. 3: die Darstellung gemäß Fig. 2, jedoch beim Herausziehen des Kolbens,
- Fig. 4: die Kanüle mit entferntem Kolben sowie Details von diesem,
- Fig. 5: die Kanüle beim Einsetzen eines abzulegenden Elements,
- Fig. 6: eine Prinzipdarstellung zur Erläuterung des Ladens der Kanüle mit einem Element sowie Schnittdarstellung von diesen,
- Fig. 7: die Kanüle mit einem Element beim Einführen des Kolbens und diese in Schnittdarstellung,
- Fig. 8: die Kanüle mit Kolben vor dem Ablegen des Elements,
- Fig. 9: die Kanüle beim Ablegen des Elementes,
- Fig. 10: die Kanüle mit Kolben nach Ablegen des Elementes,
- Fig. 11: die Kanüle mit Kolben vor Aktivierung eines Sicherheitsmechanismus,
- Fig. 12: die Kanüle mit Kolben in der Bewegung zur Verriegelung,
- Fig. 13: der mit der Kanüle bzw. deren Ansatz verriegelten Kolben,
- Fig. 14: eine weitere Ausführungsform einer Kanüle mit Kolben und Schutzhülle im Anlieferungszustand,
- Fig. 15: die Kanüle mit Kolben gemäß Fig. 14, jedoch mit entfernter Schutzhülle,
- Fig. 16: die Kanüle mit entferntem Kolben,
- Fig. 17: die Kanüle in einer Position, in der ein Element in diese eingebracht wird und der Kolben auf die Kanüle ausgerichtet ist,
- Fig. 18: die Kanüle mit Kolben beim Ablegen eines Elementes und
- Fig. 19: die Kanüle mit deren Spitze abdeckendem Kolben.

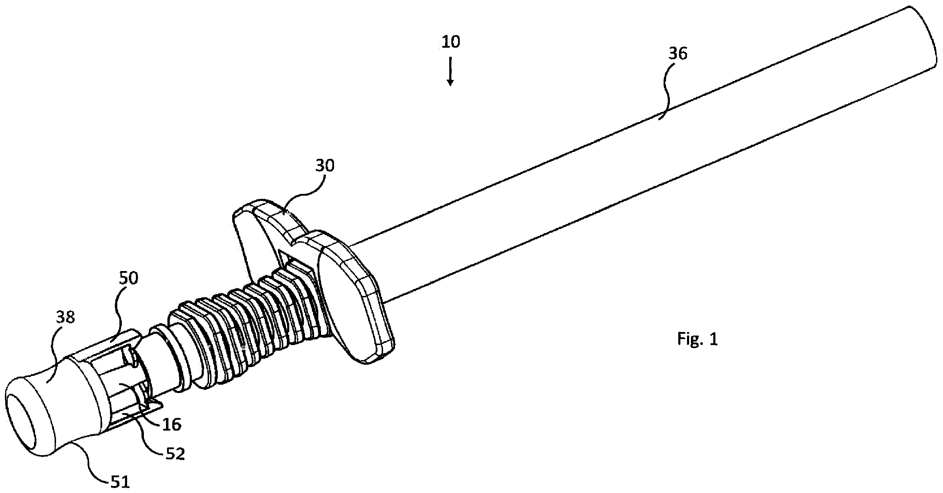

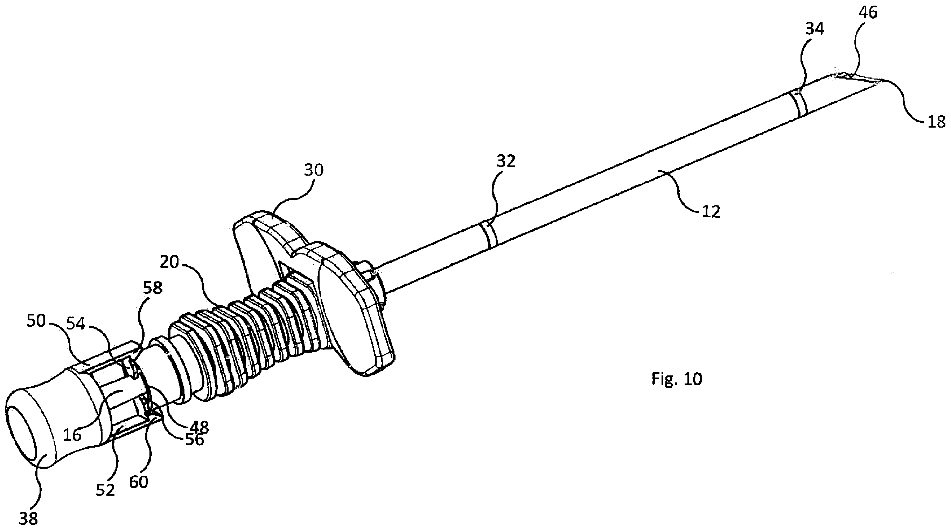

Den Figuren 1 bis 13, in denen für gleiche Elemente gleiche Bezugszeichen verwendet werden, ist eine Vorrichtung 10, insbesondere zum Ablegen von Elementen, wie Feststoffmedikament oder Implantat, wie Identifizierungs- oder Überwachungschip, zu entnehmen. Hierzu wird die Vorrichtung 10 mit einer Kanüle 12 zum Injizieren des Elements 14 in ein Lebewesen sowie ein Kolben 16 - auch Mandrin genannt - benutzt, mittels dessen das Element 14 durch die Kanüle 12 hindurch geschoben wird.

In Fig. 1 ist die Vorrichtung 10 im Anlieferungszustand dargestellt.

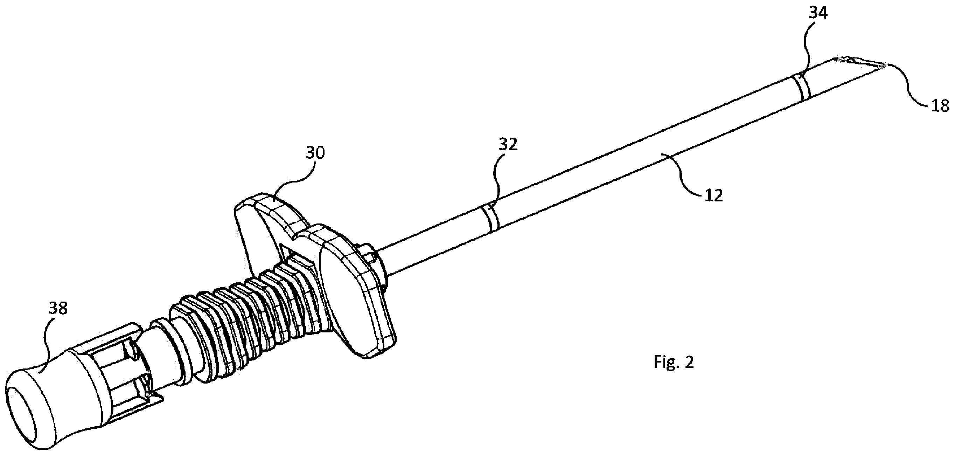

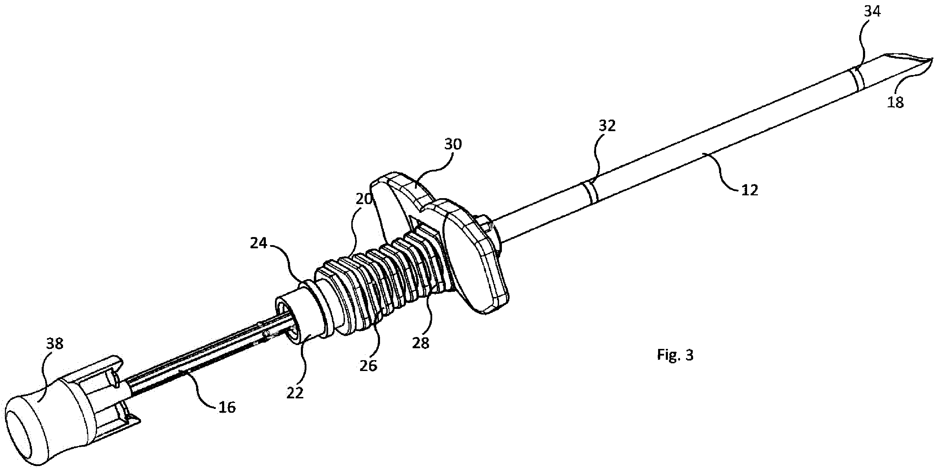

Man erkennt in Fig. 2 die Kanüle 12, die distal eine Spitze 18 aufweist und deren proximaler Bereich von einem Ansatz 20 aufgenommen ist, der aus einem hohlzylindrischen Grundkörper 22 besteht, von dessen Umfangswandung im Ausführungsbeispiel Rippen abragen, von denen einige mit den Bezugszeichen 24, 26, 28 gekennzeichnet sind (Fig. 3). Im distalen Bereich des Ansatzes 20 findet sich eine flügelartige Handhabe 30, um ein problemloses Hantieren zu ermöglichen. Insoweit weist die Kanüle 12 mit dem Ansatz 20 eine hinlänglich bekannte Konstruktion auf.

Auf der Außenfläche der Kanüle 12 können Markierungen 32, 34 vorgesehen sein, um die Eindringtiefe der Kanülenspitze 18 in ein Lebewesen zu ermitteln.

Im Anlieferungszustand ist die Kanüle 12 von einer Schutzhülle 36 umgeben, die vor der Anwendung abgezogen wird. Die Schutzhülle 36 kann z.B. über Sollbruchstellen mit der flügelartigen Handhabe 30 verbunden sein.

Der Kolben 16 weist proximal eine Handhabe 38 auf, um diesen zu erfassen damit der Kolben 16 in die Kanüle 12 hineingeschoben bzw. aus dieser herausgezogen werden kann. Letzteres erfolgt dann, wenn mittels der Kanüle 12 nacheinander mehrere Elemente abgelegt werden sollen.

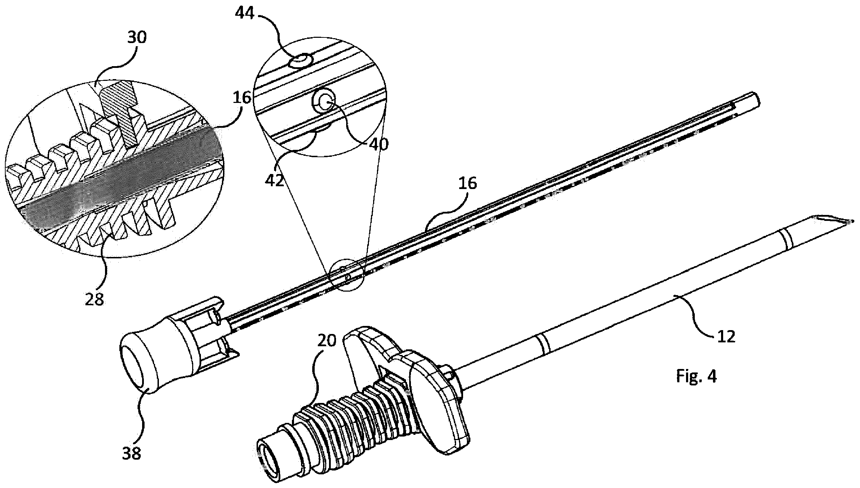

Im Abstand zu der Handhabe 38 gehen von der Umfangsfläche des Kolbens 16 mehrere Vorsprünge 40, 42, 44 aus, die vorzugsweise gleichmäßig um den Umfang herum verteilt sind. Die Vorsprünge 40, 42, 44 ermöglichen ein Halten des Kolbens 16 innerhalb der Kanüle 12 durch Reibschluss, so dass beim Transport ein unkontrolliertes Herausgleiten nicht möglich ist. Die Vorsprünge 40, 42, 44 befinden sich im proximalen Endbereich des Kolbens 16 und damit der Kanüle 12, wenn der Kolben 16 in die Kanüle 12 hineingeschoben worden ist (Fig. 4).

Ist in Fig. 1 die Vorrichtung 10 im Anlieferungszustand dargestellt, so ist in Fig. 2 die Schutzhülle 36 bereits abgezogen.

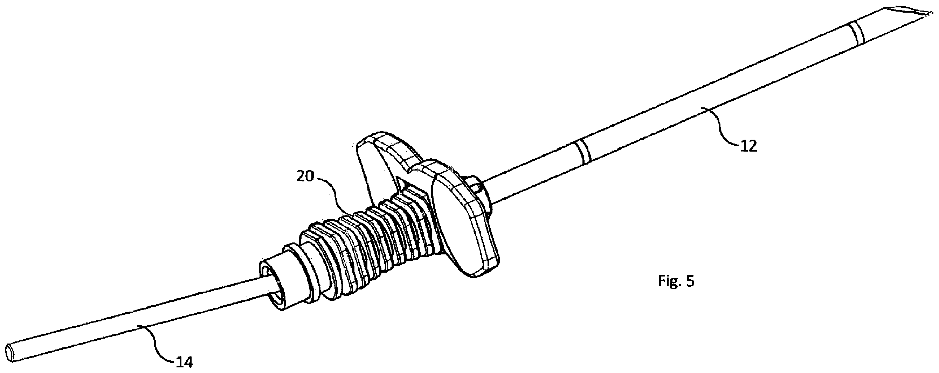

Fig. 3 verdeutlicht die Position, in der der Kolben 16 aus der Kanüle 12 herausgezogen wird, um ein Element 14 einzubringen, wie sich aus Fig. 5 prinzipiell ergibt.

In Fig. 4 sind noch einmal detailliert der Kolben 16 mit Handhabe 38 sowie die noppenartigen Vorsprünge 40, 42, 44 erkennbar, mittels der der Kolben 16 in der Kanüle 12 durch Reibschluss fixiert werden kann.

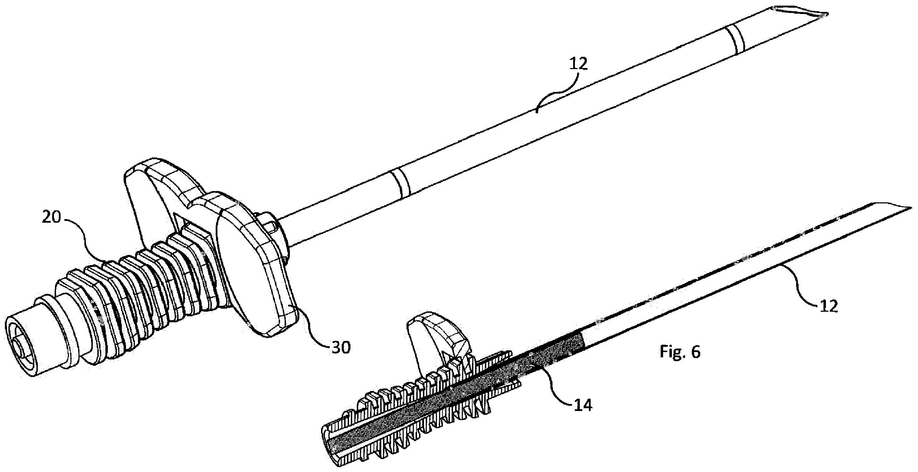

Die Fig. 5 zeigt detailliert, wie das Element 14 in die Kanüle 12 von Hand geschoben wird. Hierzu kann ein Nutzer seinen Daumen benutzen, der das Element 14 bis zur proximalen Öffnung des Ansatzes 20 hineinschiebt, so dass das Element 14 entsprechend der unteren Darstellung in Fig. 6 bereichsweise bereits innerhalb der Kanüle 12 zum Liegen kommt.

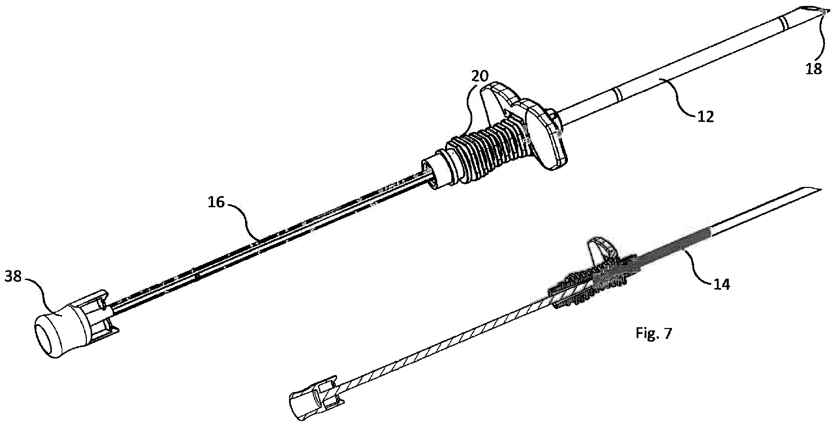

Um das Element 14 durch die Kanüle 12 hindurchzuschieben und sodann abzulegen, wird nach Einbringen des Elementes 14 gemäß Fig. 7 der Kolben 16 in die proximale Öffnung des Ansatzes 20 eingeführt, um das Element 14 in Längsrichtung der Kanüle 12 in Richtung der Spitze 18 zu verschieben.

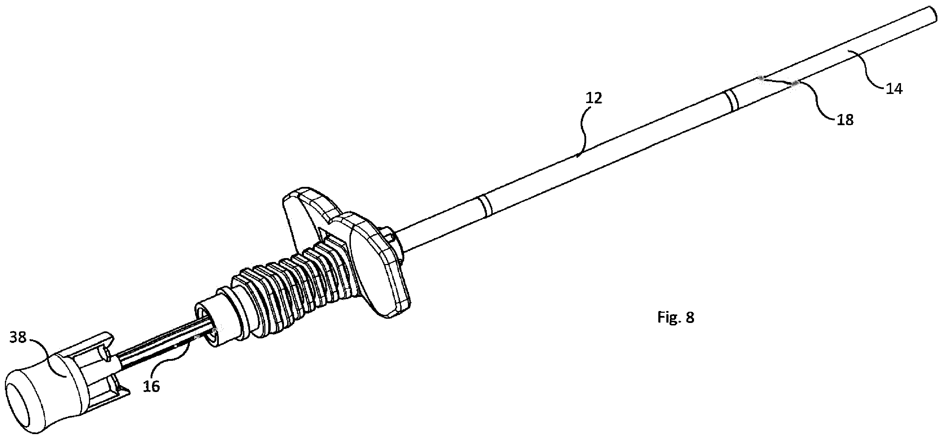

In Fig. 8 ist der Kolben 16 bereits in einem Umfang durch die Kanüle 12 hindurchgeschoben, dass das Element 14 mit seinem distalen Ende über der Spitze 18 vorsteht.

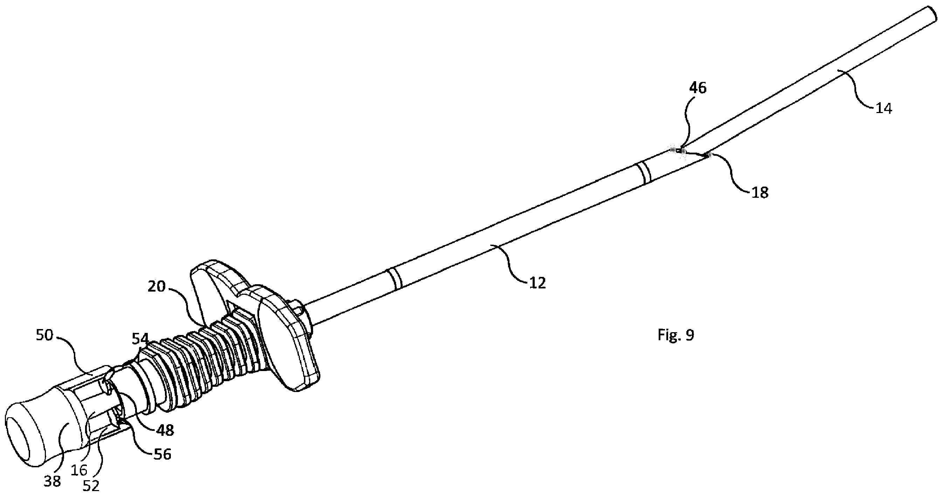

In Fig. 9 ist der Kolben 16 in einem Umfang in die Kanüle 12 hineingeschoben worden, dass das Element 14 abgelegt werden kann. Hierzu sind Länge des Kolbens 16 - im eigentlichen Sinne der Kolbenstange - und Kanüle 12 mit Ansatz 20 derart aufeinander abgestimmt, dass der Kolben 16 mit seinem distalen Ende 46 nicht über der Spitze 18 vorsteht, die von einem Schrägschliff ausgeht. In dieser Position wirkt die Handhabe 38 des Kolbens 16 mit einem von diesem ausgehenden Anschlag mit dem öffnungsseitig verlaufenden Stirnrand 48 des Ansatzes 20 zusammen. Hierzu weist die Handhabe 38 im Ausführungsbeispiel zwei diametral zur Kolbenlängsachse verlaufende und als Nasen zu bezeichnende stegartige Abschnitte 50, 52 auf, die sich in Längsachsenrichtung des Kolbens 16 und beabstandet zu diesem erstrecken, wie die Figuren selbsterklärend erläutern.

Die stegartigen Abschnitte 50, 52 gehen von einem eine Zylindergeometrie aufweisenden Basisabschnitt 51 der Handhabe 38 aus, wie aus den Zeichnungen klar ersichtlich wird. Der Basisabschnitt 51 kann in seinem Mittenbereich einen geringeren Durchmesser als in seinen Rändern aufweisen, um ein sicheres Erfassen zu ermöglichen, ohne dass ein Abrutschen erfolgt.

Die Abschnitte 50, 52, die auch als Fahnen oder Nasen zu bezeichnen sind, sind mit dem Kolben 16 über Verbindungsstege 54, 56 verbunden, also ganz allgemein über Verbindungselemente. Dabei erfolgt eine Verbindung derart, dass der Verbindungssteg 54, 56 mit der Fahne 50, 52 über eine Sollbruchstelle verbunden ist, wohingegen die Verbindung zwischen dem Verbindungssteg 54, 56 und dem Kolben 16 über eine Art Filmscharnier erfolgt. Die Verbindungsstege 54, 56 gehen dabei von hakenförmigen sich in Richtung des Kolbens 16 erstreckenden Vorsprüngen 58, 60 aus, die sich von den Enden der Fahnen oder Nasen 50, 52 in Richtung des Kolbens 16 erstrecken. Dabei weisen die hakenförmigen Vorsprünge 58, 60 einen lichten Abstand auf, der in etwa gleich dem Außendurchmesser des Ansatzes 20 im Bereich dessen proximaler Öffnung ist.

Die Erfindung wird selbstverständlich dann nicht verlassen, wenn der Verbindungssteg 54, 56 mit dem Kolben 16 über eine Sollbruchstelle und mit dem Halteelement 50, 52 über ein Filmscharnier verbunden ist.

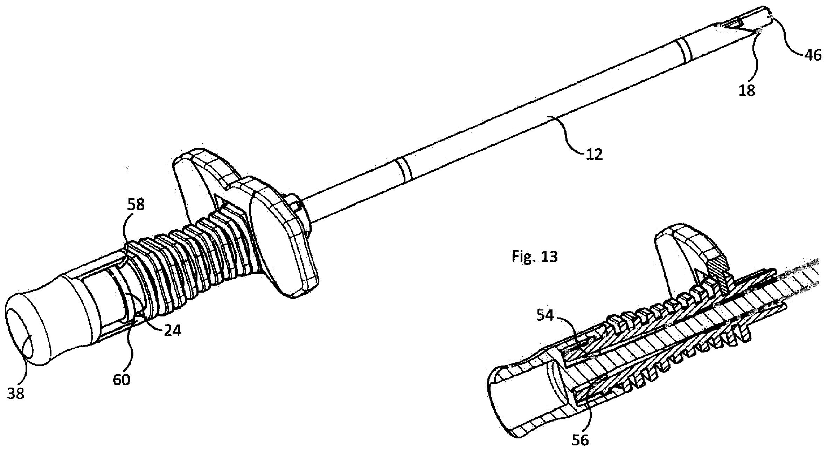

Sollen mittels der Kanüle 12 mehrfach Elemente 14 abgelegt werden, so wird nach Ablegen eines Elementes 14 der Kolben 16 zurückgezogen, um in zuvor beschriebener Art ein neues Element 14 einzubringen und sodann abzulegen. Dabei wirken die auch als Anschlagelemente zu bezeichnenden Verbindungsstege 54, 56 als Anschläge, so dass sichergestellt ist, dass die als Rastelemente dienenden stegartigen Halteelemente, also die Nasen 50, 52 in nachstehend beschriebener Art und Weise nicht mit einer der Rippen des Ansatzes 20 wechselwirken und diese hintergreifen können, wodurch ansonsten ein Verrasten des Kolbens 16 erfolgt, wie dies der Fig. 13 zu entnehmen ist.

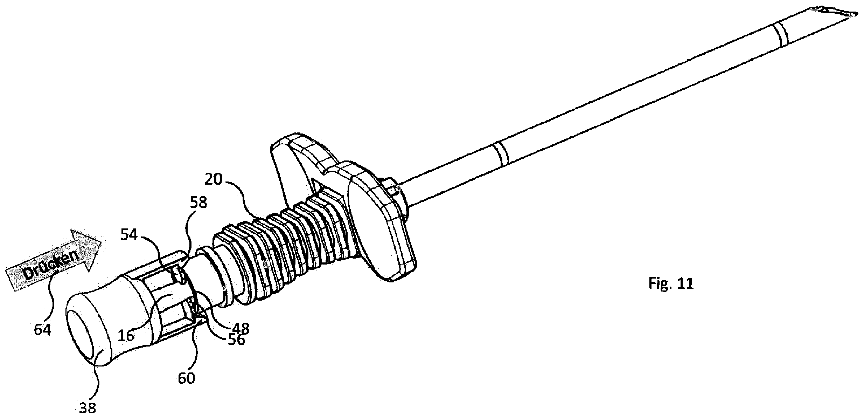

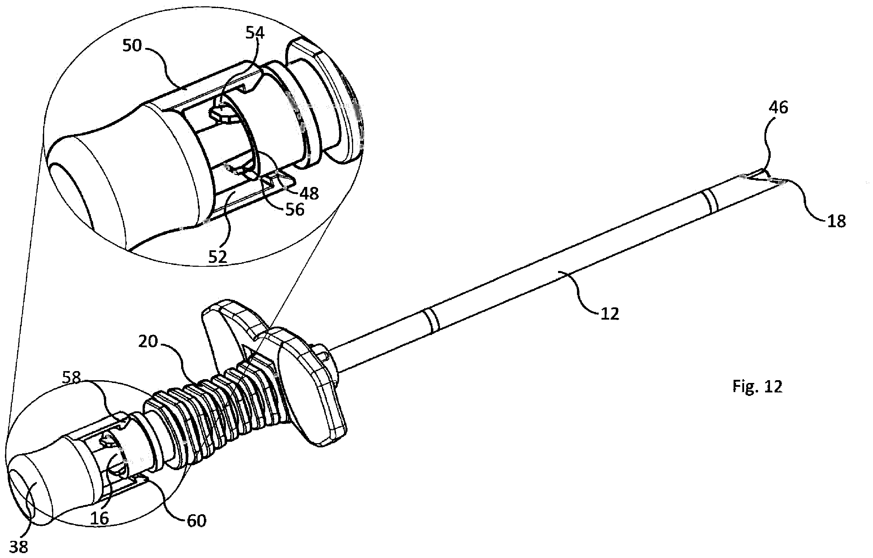

Um ein solches Verrasten zu ermöglichen, muss auf die Handhabe 38 des Kolbens 16 in Richtung des Pfeils 64 (Fig. 11) eine Kraft derart einwirken, dass die zuvor als Anschlag dienenden Verbindungsstege 54, 56 in ihren Verbindungsbereichen zu den hakenförmigen Vorsprüngen 58, 60 quasi abgeschert werden, da auf diese Bereiche der Rand der Öffnung des Ansatzes 20 einwirkt. Ungeachtet dessen verlieren sich die Verbindungsstege 54, 56 nicht, da diese erfindungsgemäß gelenkig mit dem Kolben 16 verbunden bleiben und entsprechend der Fig. 12 beim Eindringen des Kolbens 16 in den Ansatz 20 hinein, also in dessen Durchgangsöffnung, in Richtung des Kolbens 16 umgebogen werden, so dass die Stege 54, 56 bei bleibender Verbindung mit dem Kolben in dem Zwischenraum zwischen der Öffnung des Ansatzes 20 und dem Kolben 16 verbleiben, wie sich aus der Fig. 13 ergibt. Gleichzeitig können die Rasthaken 58, 60 im Ausführungsbeispiel die unterste oder erste Rippe 24 des Ansatzes 20 der Kanüle 12 hintergreifen (Fig. 13).

Ein Nutzer nimmt dies nicht nur haptisch wahr. Durch das Abbrechen der Verbindungsstege 54, 56 von den stegförmigen Halteelementen bzw. Nasen 50, 52 treten zudem wahrnehmbare Geräusche auf, die den Nutzer gleichfalls darauf hinweist, dass die Sicherheitsfunktion ausgelöst wird, um den Kolben 16 derart mittels der Nasen 50, 52 mit dem Ansatz 20 zu verrasten, dass ein unkontrolliertes Herausziehen aus der Kanüle 12 nicht möglich ist.

Zu erwähnen ist, dass die Rippen 24, 26, 28 die Erfindung nicht einschränken. Es können ein oder mehrere andere Elemente vorgesehen sein, die von dem Ansatz 20 ausgehen oder in diesem integriert sind, um ein Verrasten des Kolbens 16 bei nicht mehr zu benutzender Vorrichtung 10 sicherzustellen.

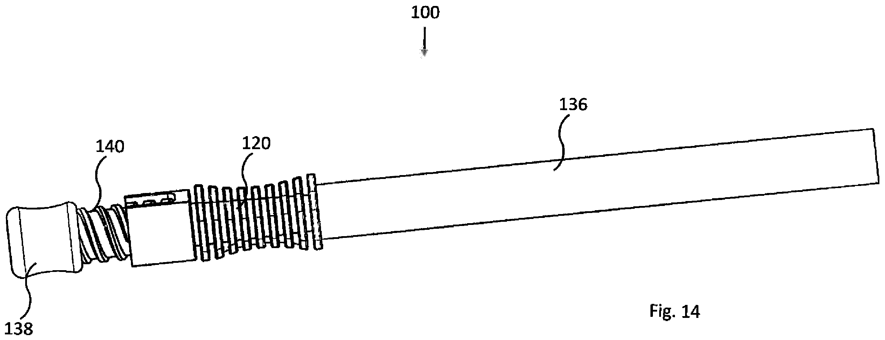

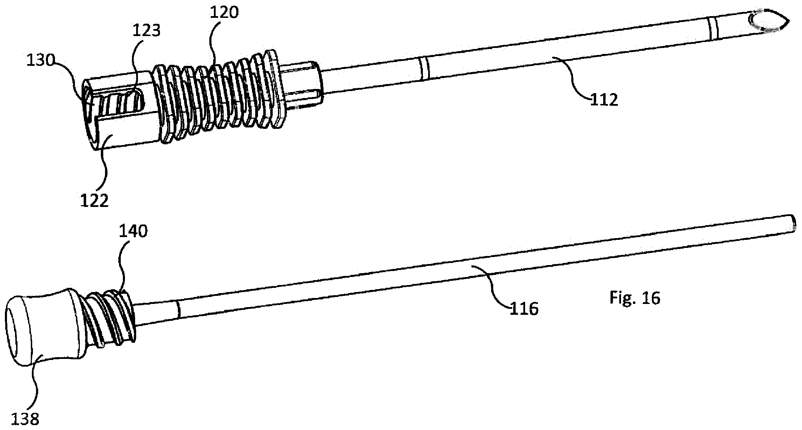

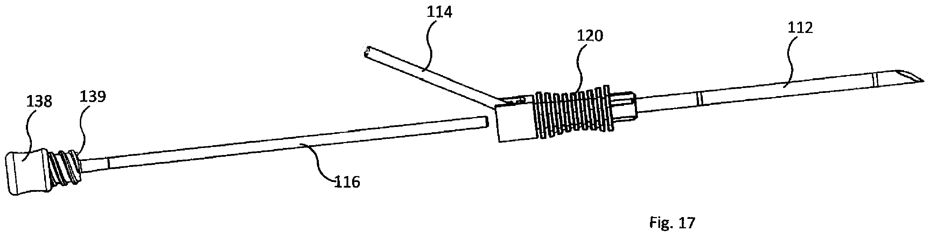

Den Figuren 14 bis 17 ist eine weitere Ausführungsform einer Vorrichtung 100 zu entnehmen, mit der, wie mit der Vorrichtung 10, ein Element 114 abgelegt werden soll, gleichzeitig sichergestellt ist, dass mit konstruktiv einfachen Maßnahmen ein Schutz gegen Verletzungen bei Nutzung der Vorrichtung 100 ausgeschlossen ist. Dabei soll im Falle, dass eine Verwendung zum Ablegen von Feststoffmedikamenten oder Implantaten erfolgen soll, die Möglichkeit geschaffen sein, mehrere Elemente 114 mit der Vorrichtung 100 nacheinander in einem Körper abzulegen.

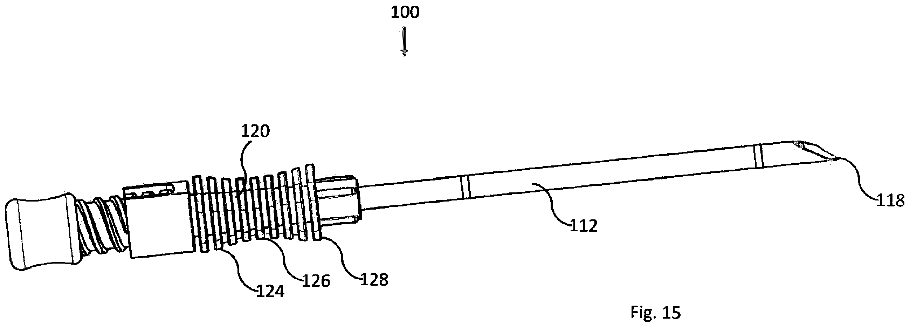

In der Figur 14 ist die Vorrichtung 100 im Anlieferungszustand und in Figur 15 nach Entfernen einer die Kanüle 112 umgebenden Schutzhülle 136 dargestellt.

Wie bei dem Ausführungsbeispiel der Figuren 1 bis 13 weist die Kanüle 112 distal eine Spitze 118 und proximal einen Ansatz 120 auf, der im Ausführungsbeispiel senkrecht zur Längsachse der Kanüle 112 verlaufende Rippen aufweist, von denen einige mit dem Bezugszeichen 124, 126, 128 gekennzeichnet sind. Selbstverständlich sind die Rippen 124, 126, 128 kein zwingendes Merkmal des Ansatzes 120.

Um im Ausführungsbeispiel ein Element 114, wie Feststoffmedikament oder Implantat, mittels der Kanüle 112 abzulegen, muss dieses durch die Kanüle 112 hindurch- und über den distalen Bereich hinausgeschoben werden. Hierzu wird ein auch als Mandrin zu bezeichnender Kolben 116 benutzt, der proximal eine Handhabe 138 aufweist, um den Kolben 116 zu erfassen und in die Kanüle 112 einführen und in deren Längsachsenrichtung verschieben zu können.

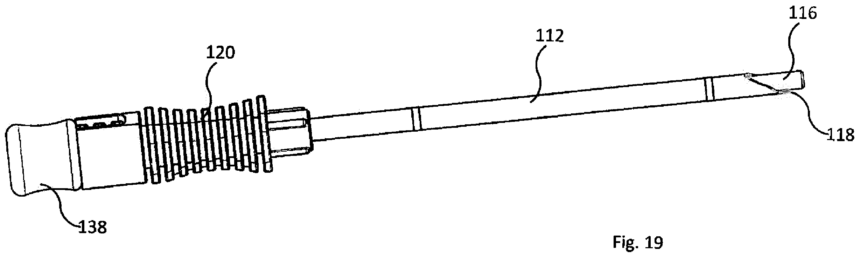

Erfindungsgemäß weist der Ansatz 120 proximal einen hohlzylindrischen Abschnitt 122 mit Innengewinde 130 auf. Ein dem Innengewinde 130 angepasstes Außengewinde 140 ist im distalen Bereich der Handhabe 138 ausgebildet, so dass die Handhabe 138 mit dem Ansatz 120 verschraubt werden kann. Dies soll dann erfolgen, wenn nach Ablegen des Feststoffmedikaments 114 eine weitere Nutzung der Kanüle 112 nicht mehr erfolgen und deren Spitze 118 vom distalem Endbereich des Kolbens 116 abgedeckt werden soll, wie dies der Figur 19 zu entnehmen ist. Dabei ist die Länge des Kolbens 116 auf die Länge der Kanüle 112 mit deren Ansatz 120 derart abgestimmt, dass dann, wenn die Handhabe 138 in den Ansatz 120 hineingeschraubt und vorzugsweise in der Endposition mit diesem verrastet ist, der distale Endbereich des Kolbens 116 über der Spitze 118 vorsteht, wie dies aus der Figur 19 offensichtlich wird.

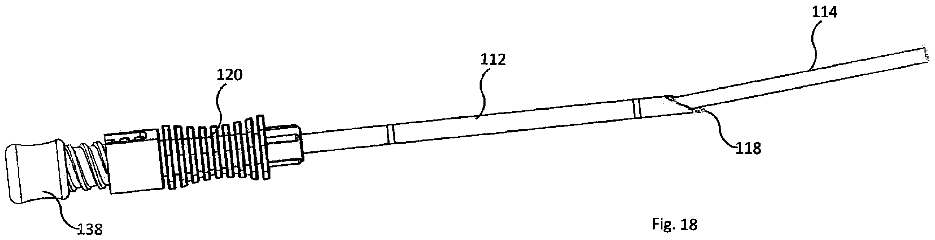

Die erfindungsgemäße Vorrichtung 100 bietet dem Nutzer zwei definierte Positionen zwischen Kolben 116 und Kanüle 112. Die erste Position ist diejenige, in der die Handhabe 138, das heißt deren distaler Randbereich 139 gegen den Gewindeanfang des Innengewindes 130 stößt, also eine Drehbewegung zwischen der Handhabe 138 und dem Ansatz 120 nicht erfolgt ist. In dieser Position, die der Figur 18 zu entnehmen ist, ist der Kolben 116 in einem Umfang innerhalb der Kanüle 112 verschoben worden, dass das Feststoffmedikament 114 abgelegt werden kann.

Die zweite Position liegt dann vor, wenn die Handhabe 138 und Ansatz 120 miteinander verschraubt sind, wobei bei vollständigem Hineinschrauben der Handhabe 138 in den Ansatz 120 zuvor ein ein Verrasten bewirkendes Überwinden eines Arretierelementes wie Vorsprungs erfolgt, so dass eine entgegengesetzte Schraubbewegung nicht mehr möglich ist mit der Folge, dass die Kanülenspitze 118 durch den Kolben 116 bedeckt bleibt und somit eine Verletzungsgefahr für den Nutzer ausgeschlossen ist.

Wie sich aus den Figuren ergibt, weist der proximale hohlzylindrische Abschnitt 122 einen in Längsachsenrichtung der Kanüle 112 verlaufenden Schlitz 123 auf, über den das Feststoffmedikament 114 eingeführt werden kann.

Zeigt das Ausführungsbeispiel die Handhabe 138 mit Außengewinde 140 und den Ansatz 120 mit Innengewinde 130, so kann selbstverständlich auch eine Konstruktion gewählt werden, bei der die Handhabe 138 einen distal verlaufenden hohlzylindrischen Abschnitt mit Innengewinde aufweist, der mit einem Außengewinde oder mit Stegen des Ansatzes 120 zusammenwirkt, um eine Schraubverbindung zu ermöglichen.

## Patentansprüche

1. Vorrichtung (10), insbesondere zum Ablegen eines Elementes (14), wie Feststoffmedikament oder Implantat, umfassend eine Kanüle (12) mit am distalen Ende vorhandener Spitze (18) und einem den proximalen Bereich der Kanüle aufnehmenden Ansatz (20), der vorzugsweise eine oder mehrere quer, insbesondere senkrecht, zur Längsachse der Kanüle verlaufende Rippen (24, 26, 28) aufweist, sowie einen abschnittsweise innerhalb der Kanüle verschiebbaren Kolben (16) mit einer Handhabe (38),
**dadurch gekennzeichnet,**
**dass** die Handhabe (38) in Längsrichtung des Kolbens (16) und beabstandet zu diesem sich erstreckende stegartige Halteelemente (50, 52) aufweist, und dass jedes Halteelement mit dem Kolben über ein Verbindungselement (54, 56) verbunden ist, das zumindest in einem seiner Endbereiche durch Wechselwirken mit dem Ansatz (20) durchtrennbar ist.

2. Vorrichtung nach Anspruch 1,
**dadurch gekennzeichnet,**
**dass** das Verbindungselement (54, 56) durchtrennbar mit dem Halteelement (50, 52) und gelenkig mit dem Kolben (16) verbunden ist oder umgekehrt.

3. Vorrichtung nach Anspruch 1 oder 2,
**dadurch gekennzeichnet,**
**dass** zur mehrfachen Nutzung der Vorrichtung (10), insbesondere zum Ablegen von Elementen (14), die Verbindungselemente (54, 56) als Anschlag mit proximalem Rand (48) des Ansatzes (20) zusammenwirken.

4. Vorrichtung nach zumindest einem der vorhergehenden Ansprüche,
**dadurch gekennzeichnet,**
**dass** das Halteelement (50, 52) einen in Richtung des Kolbens (16) sich erstreckenden Rastabschnitt (58, 60) aufweist, der vorzugsweise ein Rastelement, wie eine Rippe (24), des Ansatzes (20) hintergreift.

5. Vorrichtung nach zumindest einem der vorhergehenden Ansprüche,
**dadurch gekennzeichnet,**
**dass** bei über der Spitze (18) der Kanüle (12) vorstehendem distalen Ende des Kolbens (16) und Verrastung des Kolbens die Verbindungselemente (54, 56) von den Halteelementen (50, 52) getrennt und bei bleibender Verbindung mit dem Kolben entlang des Kolbens (16) ausgerichtet sind oder die Verbindungselemente (54, 56) von dem Kolben getrennt sind und vorzugsweise bei bleibender Verbindung mit den Halteelementen (50, 52) entlang dieser ausgerichtet sind.

6. Vorrichtung nach zumindest Anspruch 1,
**dadurch gekennzeichnet,**
**dass** die Verbindungselemente (54, 56) sowohl von dem Kolben (16) als auch von den Halteelementen (50, 52) durch Wechselwirken mit dem Ansatz (20) durchtrennbar sind.

7. Vorrichtung nach zumindest einem der vorhergehenden Ansprüche,
**dadurch gekennzeichnet,**
**dass** das Verbindungselement (54, 56) mit dem Rastabschnitt (58, 60) verbunden ist.

8. Vorrichtung nach zumindest einem der vorhergehenden Ansprüche,
**dadurch gekennzeichnet,**
**dass** der Ansatz (20) proximal als Hohlzylinderabschnitt mit einem Außendurchmesser D ausgebildet ist, und dass lichter Abstand von zwei in Bezug zur Kolbenlängsachse diametral gegenüberliegenden Rastabschnitten (58, 60) gleich D oder in etwa gleich D ist.

9. Vorrichtung nach zumindest einem der vorhergehenden Ansprüche,
**dadurch gekennzeichnet,**
**dass** die Halteelemente (50, 52) von einem Basisabschnitt (51) der Handhabe (38) des Kolbens (16) ausgehen, der eine zylinderförmige Außengeometrie in seinen Endbereichen aufweist, wobei vorzugsweise die Halteelemente (50, 52) außenseitig fluchtend in Umfangsfläche des Basisabschnitts (51) übergehen.

10. Vorrichtung nach zumindest einem der vorhergehenden Ansprüche,
**dadurch gekennzeichnet,**
**dass** das Verbindungselement (54, 56) über ein Filmscharnier mit dem Kolben (16) oder dem Halteelement (50, 52) verbunden ist.

11. Vorrichtung (100), insbesondere zum Ablegen eines Elementes (114), wie Feststoffmedikament oder Implantat, umfassend eine Kanüle (112) mit am distalen Ende vorhandener Spitze (118) und einem den proximalen Bereich der Kanüle aufnehmenden Ansatz (120), der gegebenenfalls eine oder mehrere quer, insbesondere senkrecht, zur Längsachse der Kanüle verlaufende Rippen (124, 126, 128) aufweist, sowie einen abschnittsweise innerhalb der Kanüle verschiebbaren Kolben (116) mit einer Handhabe (138),
wobei
die Handhabe (138) mit dem Ansatz (120) über eine Schraubverbindung verbindbar ist, **dadurch gekennzeichnet, dass** die Länge des Kolbens (116) auf Länge der Kanüle (112) derart abgestimmt ist, dass in einer ersten Position, in der bei fehlender Verschraubung, jedoch Kontaktierung des Ansatzes und der Handhabe die Spitze (118) von dem Kolben unbedeckt ist, und dass in einer zweiten Position der Ansatz durch Hineinschrauben der Handhabe in den Ansatz und Überwinden eines Arretierelements verrastet sind, wobei der distale Endbereich des Kolbens die Spitze zur Unterbindung einer Verletzungsgefahr durch die Spitze abdeckt und eine entgegengesetzte Schraubbewegung ausgeschlossen ist.

12. Vorrichtung nach Anspruch 11,
**dadurch gekennzeichnet,**
**dass** die Handhabe (138) ein Außengewinde (140) aufweist, das mit einem im Ansatz (120) vorhandenen Innengewinde (130) wechselwirkt oder dass die Handhabe (138) ein Innengewinde aufweist, das entweder mit von dem Ansatz (120), insbesondere von dessen proximalem Randbereich, abragenden Stegen, wie Luer-Lock-Stege, oder mit einem Außengewinde des Ansatzes wechselwirkt.

13. Vorrichtung nach zumindest einem der vorhergehenden Ansprüche,
**dadurch gekennzeichnet,**
**dass** von dem Kolben (16), vorzugsweise in dessen proximalem Bereich, zumindest ein einen Reibschluss mit Innenfläche der Kanüle (12) ermöglichender Vorsprung (40, 42, 44) abragt, insbesondere gleichmäßig um die Umfangswandung verteilt mehrere Vorsprünge (40, 42, 44) von dem Kolben (16) abragen.

14. Vorrichtung nach zumindest einem der vorhergehenden Ansprüche,
**dadurch gekennzeichnet,**
**dass** der Kolben (16) mit der Handhabe (38) ein Kunststoffspritzgussteil ist.

15. Vorrichtung nach Anspruch 11,
**dadurch gekennzeichnet,**
**dass** das Arretierelement ein Vorsprung, insbesondere in Form eines Absatzes, ist.

## Claims

1. A device (10), in particular for depositing an element (14), such as a solid medicament or implant, comprising a cannula (12) with a tip (18) present at the distal end and an extension (20) accommodating the proximal region of the cannula, which extension preferably has one or more ribs (24, 26, 28) extending transversely of, in particular perpendicularly to, the longitudinal axis of the cannula, and a plunger (16) with a handle (38) which is displaceable in part within the cannula,
**characterised in that**
the handle (38) has tab-like retaining elements (50, 52) in the longitudinal direction of the plunger (16) and extending spaced therefrom, and **in that** each retaining element is connected to the plunger via a connecting element (54, 56) which is severable at least in one of the end regions thereof by interaction with the extension (20).

2. The device according to Claim 1,
**characterised in that**
the connecting element (54, 56) is severably connected to the retaining element (50, 52) and articulatedly connected to the plunger (16) or vice versa.

3. The device according to Claim 1 or 2,
**characterised in that**
for multiple use of the device (10), in particular for depositing elements (14), the connecting elements (54, 56) interact as a limit stop with the proximal edge (48) of the extension (20).

4. The device according to at least one of the preceding claims,
**characterised in that**
the retaining element (50, 52) has a latching portion (58, 60) extending in the direction of the plunger (16), whereby the latching portion (58, 60) preferably engages behind a latching element, such as a rib (24), of the extension (20).

5. The device according to at least one of the preceding claims,
**characterised in that,**
with the distal end of the plunger (16) projecting beyond the tip (18) of the cannula (12) and the plunger latched, the connecting elements (54, 56) are separated from the retaining elements (50, 52) and, while remaining connected to the plunger, are oriented along the plunger (16), or the connecting elements (54, 56) are separated from the plunger and, preferably while remaining connected to the retaining elements (50, 52), are oriented therealong.

6. The device according to at least Claim 1,
**characterised in that**
the connecting elements (54, 56) are severable both from the plunger (16) and from the retaining elements (50, 52) by interaction with the extension (20).

7. The device according to at least one of the preceding claims,
**characterised in that**
the connecting element (54, 56) is connected to the latching portion (58, 60).

8. The device according to at least one of the preceding claims,
**characterised in that**
the extension (20) is proximally formed as a hollow-cylindrical portion with an external diameter D, and **in that** the clearance between two latching portions (58, 60) diametrically opposed with regard to the longitudinal axis of the plunger is equal or approximately equal to D.

9. The device according to at least one of the preceding claims,
**characterised in that**
the retaining elements (50, 52) originate from a base portion (51) of the handle (38) of the plunger (16) which has a cylindrical outer geometry in its end regions, whereby preferably the retaining elements (50, 52) on the outside transition flush into the circumferential surface of the base portion (51).

10. The device according to at least one of the preceding claims,
**characterised in that**
the connecting element (54, 56) is connected via a film hinge to the plunger (16) or to the retaining element (50, 52).

11. A device (100), in particular for depositing an element (114), such as a solid medicament or implant, comprising a cannula (112) with a tip (118) present at the distal end and an extension (120) accommodating the proximal region of the cannula, which extension optionally has one or more ribs (124, 126, 128) extending transversely of, in particular perpendicularly to, the longitudinal axis of the cannula, and a plunger (116) with a handle (138) which is displaceable in part within the cannula, whereby the handle (138) is connectable to the extension (120) via a screw connection,
**characterised in that**
the length of the plunger (116) is matched with the length of the cannula (112) in such a way that in a first position, in which in the absence of a screw fastening but with the extension and the handle in contact, the tip (118) of the plunger is uncovered, and that in a second position, in which the extension and the handle are screwed together, and latched together by overcoming a locking element, wherein the distal end region of the plunger covers the tip to prevent any risk of injury by the tip and an opposing screw movement is no longer possible.

12. The device according to Claim 11,
**characterised in that**
the handle (138) has an external thread (140) which interacts with an internal thread (130) present in the extension (120), or **in that** the handle (138) has an internal thread which interacts either with tabs, such as Luer Lock tabs, projecting out from the extension (120), in particular from the proximal edge region thereof, or with an external thread of the extension.

13. The device according to at least one of the preceding claims,
**characterised in that**
at least one projection (40, 42, 44) which enables frictional engagement with the inner surface of the cannula (12) projects out from the plunger (16), preferably in the proximal region thereof, especially a plurality of projections (40, 42, 44) uniformly distributed around the circumferential wall project out from the plunger (16).

14. The device according to at least one of the preceding claims,
**characterised in that**
the plunger (16) with the handle (38) is a plastics injection moulding.

15. The device according to at least one of the preceding claims,
**characterised in that**
the locking element is a projection, especially in form of a shoulder.

## Revendications

1. Dispositif (10), notamment pour déposer un élément (14), tel qu'un médicament solide ou un implant, comprenant une canule (12) avec une pointe présente sur l'extrémité distale (18) et une rallonge (20) accueillant la zone proximale de la canule, qui présente une ou plusieurs nervures (24, 26, 28) s'étendant transversalement, de préférence perpendiculairement à l'axe longitudinal de la canule, ainsi qu'un piston (16) avec une poignée (38), déplaçable partiellement à l'intérieur de la canule,
**caractérisé en ce**
**que** la poignée (38) présente des éléments de maintien (50, 52) sous forme de barrettes, s'étendant dans le sens longitudinal du piston (16) et écartés de celui-ci, et que chaque élément de maintien est relié au piston via un élément de liaison (54, 56) qui est séparable au moins à une de ses extrémités, par interaction avec la rallonge (20).

2. Dispositif selon la revendication 1,
**caractérisé en ce**
**que** l'élément de liaison (54, 56) est relié de manière séparable à l'élément de maintien (50, 52) et de manière articulée au piston (16) ou inversement.

3. Dispositif selon la revendication 1 ou 2,
**caractérisé en ce**
**que** les éléments de liaison (54, 56) interagissent comme butée avec le bord proximal (48) de la rallonge (20), pour une utilisation réitérée du dispositif (10), notamment pour déposer des éléments (14).

4. Dispositif selon au moins l'une des revendications précédentes,
**caractérisé en ce**
**que** l'élément de maintien (50, 52) présente une section d'encliquetage (58, 60) s'étendant en direction du piston (16), qui s'accroche par l'arrière à un élément d'encliquetage, telle qu'une nervure (24) de la rallonge (20).

5. Dispositif selon au moins l'une des revendications précédentes,
**caractérisé en ce**
**qu'**au niveau de l'extrémité distale du piston (16), saillant de la pointe (18) de la canule (12) et lors de l'encliquetage du piston, les éléments de liaison (54, 56) sont séparés des éléments de maintien (50, 52) et qu'ils sont, lors de liaison persistante avec le piston, orientés le long du piston (16) ou que les éléments de liaison (54, 56) sont séparés du piston et sont orientés, en cas de liaison persistante avec les éléments de maintien (50, 52), de préférence le long de ceux-ci.

6. Dispositif selon au moins la revendication 1,
**caractérisé en ce**
**que** les éléments de liaison (54, 56) sont séparables aussi bien du piston (16) que des éléments de maintien (50, 52) par interaction avec la rallonge (20).

7. Dispositif selon au moins l'une des revendications précédentes,
**caractérisé en ce**
**que** l'élément de liaison (54, 56) est relié à la section d'encliquetage (58, 60).

8. Dispositif selon au moins l'une des revendications précédentes,
**caractérisé en ce**
**que** la rallonge (20) est conçue côté proximal comme section à cylindre creux avec un diamètre extérieur D, et que l'écart utile entre deux sections d'encliquetage (58, 60) diamétralement opposées par rapport à l'axe longitudinal du piston est égal ou presque égal à D.

9. Dispositif selon au moins l'une des revendications précédentes,
**caractérisé en ce**
**que** les éléments de maintien (50, 52) partent d'une section de base (51) de la poignée (38) du piston (16), qui présente une géométrie extérieure de forme cylindrique à ses extrémités, sachant que de préférence, les éléments de maintien (50, 52) se prolongent côté extérieur alignés à la surface circonférentielle de la section de base (51).

10. Dispositif selon au moins l'une des revendications précédentes,
**caractérisé en ce**
**que** l'élément de liaison (54, 56) est relié au piston (16) ou à l'élément de maintien (50, 52) via une charnière-film.

11. Dispositif (100), notamment pour déposer un élément (114), tel qu'un médicament solide ou un implant, comprenant une canule (112) avec une pointe présente sur l'extrémité distale (118) et une rallonge (120) accueillant la zone proximale de la canule, qui présente une ou plusieurs nervures (124, 126, 128) s'étendant transversalement, notamment perpendiculairement à l'axe longitudinal de la canule, ainsi qu'un piston (116) avec une poignée (138), déplaçable partiellement à l'intérieur de la canule,
sachant que la poignée (138) est reliable à la rallonge (120) via un raccord vissable,
**caractérisé en ce**
**que** la longueur du piston (116) est adaptée à la longueur de la canule (112) de sorte que dans une première position dans laquelle en cas d'absence de vissage, mais avec contact avec la rallonge et la poignée, la pointe (118) n'est pas recouverte par le piston et que, dans une seconde position, la rallonge est encliquetage par vissage de la poignée dans la rallonge et dépassement d'un élément de blocage, sachant que l'extrémité distale du piston recouvre la pointe pour prévenir un risque de blessure par la pointe et qu'un mouvement de vissage dans le sens opposé est exclu.

12. Dispositif selon la revendication 11,
**caractérisé en ce**
**que** la poignée (138) présente un filetage extérieur (140) qui interagit avec un filetage intérieur (130) présent dans la rallonge (120) ou que la poignée (138) présente un filetage intérieur qui interagit soit avec des barrettes, telles que des barrettes Luer-Lock, saillant de la rallonge (120), notamment de son bord proximal soit avec un filetage extérieur de la rallonge.

13. Dispositif selon au moins l'une des revendications précédentes,
**caractérisé en ce**
**qu'**au moins une saillie (40, 42, 44) permettant une liaison par adhérence avec la surface intérieure de la canule (12) dépasse du piston (16), de préférence dans sa zone proximale, notamment que plusieurs saillies (40, 42, 44) réparties uniformément autour de la paroi circonférentielle dépassent du piston (16).

14. Dispositif selon au moins l'une des revendications précédentes,
**caractérisé en ce**
**que** le piston (16) avec la poignée (38) est une pièce en plastique moulée par injection.

15. Dispositif selon la revendication 11,
**caractérisé en ce**
**que** l'élément de blocage est une saillie, notamment sous forme d'une rallonge.
